(19) Europäisches Patentamt
European Patent Office
Office européen des brevets

(11) Publication number: 0 153 994
B1

(12) **EUROPEAN PATENT SPECIFICATION**

(45) Date of publication of patent specification: **10.08.88**

(51) Int. Cl.$^4$: **C 07 D 217/14,**
C 07 D 217/16, A 61 K 31/47

(21) Application number: **84112854.9**

(22) Date of filing: **25.10.84**

(54) **(Bis(substituted methyl)-methyl)-isoquinoline derivatives, process for their preparation and pharmaceutical compositions containing them.**

(30) Priority: **25.10.83 HU 365383**

(43) Date of publication of application:
**11.09.85 Bulletin 85/37**

(45) Publication of the grant of the patent:
**10.08.88 Bulletin 88/32**

(84) Designated Contracting States:
**AT BE CH DE FR GB IT LI LU NL SE**

(58) References cited:
**DD-A- 121 321**
**US-A-3 823 148**

**CHEMISCHE BERICHTE, 102, 1969, Weinheim/Bergstr. R. HUISGEN "1,3-Dipolare Cycloadditionen, XLVII" pages 915-925**

(73) Proprietor: **RICHTER GEDEON VEGYESZETI GYAR R.T.**
**Gyömröi ut 19-21**
**H-1103 Budapest X (HU)**

(72) Inventor: **Bernáth, Gábor, Dr. Dipl.-Chem.**
**8, Mérei u.**
**Szeged (HU)**
Inventor: **Kóbor, Jenö, Dr.**
**7, Jósika u.**
**Szeged (HU)**
Inventor: **Fülöp, Ferenc, Dr. Dipl.-Chem.**
**3/B, Hont F. u.**
**Szeged (HU)**
Inventor: **Motika, Gábor, Dr. Dipl.-Chem.**
**18/A, Vajda u.**
**Szeged (HU)**
Inventor: **Sohajda, Attila, Dipl.-Pharm.**
**34, Kodály Z. u.**
**Kisvárda (HU)**
Inventor: **Ezer, Elemér, Dipl.-Chem.**
**6-8, Lupény u.**
**Budapest II (HU)**
Inventor: **Hajós, György, Dr. Dipl.-Pharm.**
**59, Gábor Aron u.**
**Budapest II (HU)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 153 994 B1**

(72) Inventor: **Pálosi, Eva, Dr.**
**21, Vend u.**
**Budapest II (HU)**
Inventor: **Dénes, László, Dr. Dipl.-Pharm.**
**19, Szél u.**
**Budapest III (HU)**
Inventor: **Szporny, László, Dr.**
**7, Szabolcska M. u.**
**Budapest XI (HU)**

(74) Representative: **Beszédes, Stephan G. Dr.**
**Münchener Strasse 80a Postfach 1168**
**D-8060 Dachau (DE)**

**Description**

The invention is concerned with new [bis(substituted methyl)-methyl]-isoquinoline derivatives as well as with a process for preparing them and pharmaceutical compositions, particularly with immunsuppressive, antidepressive, analgesic, antipyretic, antihypoxial, gastric acid inhibiting, anticonvulsive and antitetrabenazine activity, containing these compounds.

According to Chem.Ber *102,* 915 (1969) 1-[bis(hydroxy-methyl]-isoquinoline was prepared from 1-methyl-isoquinoline with formaldehyde. The compound was afforded in a yield of 60%, after boiling for 40 hours. The only reaction of the compound obtained examined in this article was its hydrogenation in the presence of platinum oxide catalyst, which resulted in the corresponding 5,6,7,8-tetrahydroisoquinoline in a 30% yield. No N– and/or O-substituted derivatives of these compounds were prepared, and it was neither disclosed nor suggested that the compounds or their derivatives would be pharmaceutically active.

Furthermore from US patent 3 823 148 there have been known 1,2,3,4-tetrahydroisoquinoline derivatives which have or may have, respectively, in the position 2 a hydrogen atom and in the position 6 and 7 hydroxy groups or methoxy radicals, respectively, and may have in the position 1 only a biphenylyl radical, an adamantyl radical, a suphonamidophenyl radical, a diaminophenyl radical, a benzyl radical with an amino substituent on the benzene ring or a phenylethyl radical substituted on the benzene ring. In this publication also the pharmacological, such as antidepressant and spasmolytic, activity of its compounds has been described.

Moreover from DDR patent 121,321 there have been known 1,2,3,4-tetrahydroisoquinoline derivatives which may have in the position 2 a hydrogen atom, an alkyl or aralkyl radical or an acyl radical and may have in the position 1 only hydrogen or an alkyl or aryl radical. In this publication also the pharmacological activity, such as the activity against diseases of the coronary vessels and against heart-arrhythmias of those compounds in which in the position 2 is hydrogen or an alkyl or aralkyl radical has been described.

The problem underlying the invention is to create novel [bis(substituted methyl)-methyl]-isoquinoline derivatives having valuable pharmacological effects and further serving as intermediates for preparing other isoquinoline derivatives having valuable pharmacological properties, a process for preparing them and pharmaceutical compositions containing them.

The above surprisingly has been attained by the invention.

The subject-matter of the invention are [bis(substituted methyl)-methyl]-isoquinoline derivatives of the formula (I)

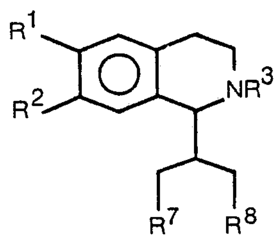

(I),

wherein

$R^1$ and $R^2$ represent hydroxyl or alkoxy having from 1 to 6 carbon atoms,

$R^3$ is hydrogen, aralkyl having from 1 to 4 carbon atoms in the alkyl moiety or a

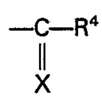 group,

in which $R^4$ is alkyl having from 1 to 5 carbon atoms or phenyl, optionally substituted by one or more alkyl having from 1 to 4 carbon atoms, alkoxy having from 1 to 4 carbon atoms, halogen and/or nitro, or is —NH-phenyl, X is oxygen or sulfur, $R^7$ is hydroxyl, halogen or an

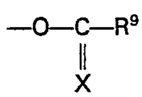 group,

$R^8$ is halogen, alkoxy having from 1 to 4 carbon atoms or an

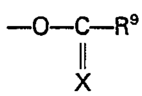 group,

in which X has the same meanings as defined above, and $R^9$ independently from $R^4$ may have the same meanings as defined for $R^4$, or is an —NH($C_{1-4}$-alkyl) or $C_{3-6}$-cycloalkyl group,

and salts thereof.

In the above formula, in the definition of $R^1$, $R^2$, $R^4$ and $R^8$ the term "alkoxy" as such or part of another group is used to refer to straight chained or branched alkoxy groups, e.g. methoxy, ethoxy, n- or isopropoxy, n-, sec.- or tert.-butoxy, n- or isopentoxy, n- or isohexoxy groups, depending on the limitation given for the number of carbon atoms.

Advantageously the alkoxy which may be represented by $R^1$ and/or $R^2$ are such having from 1 to 4 carbon atoms.

Preferably $R^1$ and $R^2$ represent identical groups.

The term "alkyl" as such or as part of other groups is used to refer to straight-chained or branched alkyl groups, e.g. methyl, ethyl, n-propyl, isopropyl, n-butyl, isobutyl, sec.-butyl, tert.-butyl, n-pentyl, isopentyl, n-hexyl and isohexyl groups, depending on the limitations given for the number of carbon atoms.

Advantageously the alkyl which may be represented by $R^4$ is such having from 1 to 4 carbon atoms.

The term "aralkyl containing from 1 to 4 carbon atoms in the alkyl moiety" in the definition of $R^3$ preferably represents a phenyl-$C_{1-4}$-alkyl group; a further example is a diphenyl-$C_{1-4}$-alkyl group.

Preferably the alkyl which may be represented by $R^4$ and/or $R^9$ or constituting the alkyl moiety of the aralkyl which may be represented by $R^3$ or by which the phenyl which may be represented by $R^4$ and/or $R^9$ may be substituted and/or the alkoxy which may be represented by $R^1$ and/or $R^2$ and/or $R^8$ and/or the alkoxy by which the phenyl which may be represented by $R^4$ and/or $R^9$ may be substituted is or are, respectively, such having 1 or 2 carbon atoms and the alkyl of the —NH($C_{1-4}$-alkyl) group which may be represented by $R^9$ is such having 3 or 4 carbon atoms.

As [an] alkyl group(s) which may be represented by $R^4$ and/or $R^9$ [the] methyl group(s) is or are, respectively, particularly preferred.

As an aralkyl group which may be represented by $R^3$ the benzyl group is particularly preferred.

Preferably the number of the substituents by which the phenyl group which may be represented by $R^4$ and/or $R^9$ may be substituted is 1 to 3.

As [an] alkyl group(s) by which the phenyl group which may be represented by $R^4$ and/or $R^9$ may be substituted [the] methyl group(s) is or are, respectively, particularly preferred.

As [an] alkoxy group(s) by which the phenyl group which may be represented by $R^4$ and/or $R^9$ may be substituted [the] methoxy group(s) is or are, respectively, particularly preferred.

As an alkoxy group which may be represented by $R^8$ the methoxy group is particularly preferred.

As a —NH($C_{1-4}$-alkyl) group which may be represented by $R^9$ the —NH(n-butyl) group is particularly preferred.

The term "halogen" is used to include all halogen atoms, i.e. fluorine, chlorine, bromine and iodine, preferably chlorine, bromine and iodine.

Preferably the halogen(s) which may be represented by $R^7$ and/or $R^8$ and/or by which the phenyl which may be represented by $R^4$ and/or $R^9$ may be substituted is or are, respectively, chlorine and/or bromine, particularly chlorine.

Furthermore it is preferred that the cycloalkyl which may be represented by $R^9$ is such having 5 or 6, particularly 6 [cyclohexyl], carbon atoms.

Preferably X represents oxygen.

Compounds of the formula (I), in which $R^7$ is hydroxyl, $R^8$ is an

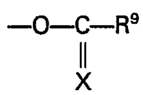

group (the other substituents are as defined above) are particularly preferred.

Extraordinarily preferred compounds according to the invention are 1-[1'-(hydroxymethyl)-1'-(benzoyloxymethyl)-methyl]-6,7-dimethoxy-1,2,3,4-tetrahydroisoquinoline hydrochloride and 1-[1'-(hydroxymethyl)-1'-(m,p,m'-trimethoxy-benzoyloxymethyl)-methyl]-6,7-diethoxy-1,2,3,4-tetrahydroisoquinoline hydrochloride.

All compounds according to the invention differ from all compounds of US patent 3,823,148 in that the former had in the position 1 a bis-(substituted methyl)-methyl radical substituted by 2 substituents $R^7$ and $R^8$ with the above definitions, whereas the latter have in the position 1 only entirely other radicals, namely a biphenylyl radical, an adamantyl radical, a sulphonamidophenyl radical, a diaminophenyl radical, a benzyl radical with an amino substituent on the benzene ring or a phenyläthyl radical substituted on the benzene ring.

All compounds according to the invention differ from all compounds according to DDR patent 121,321 in that the former have in the position 1 a bis-(substituted methyl)-methyl radical substituted by 2 substituents $R^7$ and $R^8$ with the above definitions, whereas the latter may have in the position 1 only hydrogen or an alkyl or aryl radical.

The subject-matter of the invention is also a process for preparing the compounds according to the invention which is characterized in reacting a [bis(hydroxymethyl)-methyl]-isoquinoline derivative of the formula (II),

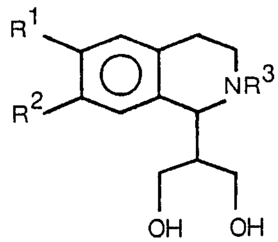

(II),

wherein $R^1$, $R^2$ and $R^3$ are as defined above,

$a_1$) with a halogenating agent to prepare compounds of formula (I), in which $R^7$ and $R^8$ both represent halogen, and, if desired, selectively alkoxylating a compound obtained, to yield compounds of formula (I), in which $R^7$ is-halogen and $R^8$ is alkoxy having from 1 to 4 carbon atoms, or

$a_2$) with an acylating agent suitable for the introduction of an

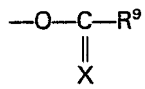 group,

to prepare compounds of formular (I), in which $R^7$ and $R^8$ both represent an

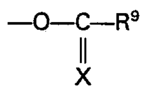 group,

in which $R^9$, X, $R^1$, $R^2$ and $R^3$ are as defined above, or

b) converting an N-substituted [bis(hydroxymethyl)-methyl]-isoquinoline derivative of the formula (III),

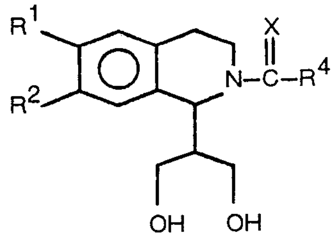

(III),

in which $R^1$, $R^2$, $R^4$ and X are as defined above, into a corresponding O-acyl derivative by N→O acyl migration, in the presence of a catalytic amount of an acid and, if desired, halogenating a compound of formula (IV) obtained,

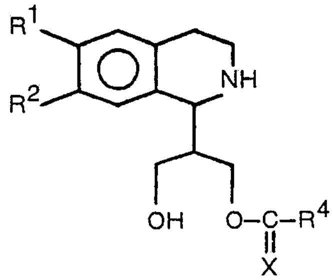

(IV),

in which $R^1$, $R^2$, $R^4$ and X are as defined above, or a salt thereof and, if desired, splitting off the acyl or thioacyl group from a compound of formula (V) obtained,

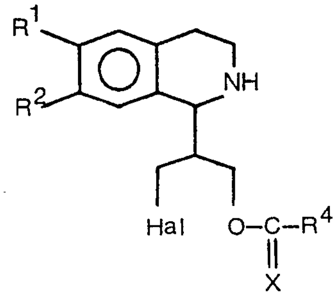

(V),

in which $R^1$, $R^2$, $R^4$ and X are as defined above and Hal represents halogen, or, if desired, converting a compound of formula (V) obtained, in which in which $R^1$, $R^2$, $R^4$ and Hal are as defined above, into a corresponding compound of formula (I), in which $R^3$ represents an

$$-\underset{\underset{X}{\|}}{C}-R^4 \text{ group,}$$

by O→N acyl migration, in an alkaline medium and, if desired, from the compounds, in which $R^7$ and/or $R^8$ is acyl, the acyl group(s) can be split off, for example by acid treatment, to yield compounds containing hydroxyl group(s) in the respective position(s), and, if desired, the acyl group(s) can be replaced by other acyl group(s) as well, and/or, if desired, converting a compound of formula (I) obtained into a salt thereof or converting a salt of a compound of formula (I) obtained into the corresponding free base of formula (I) and/or into another salt.

The isoquinoline derivatives of the formula (II), which are used as starting compounds in variants $a_1$) and $a_2$) of the process according to the invention, are new compounds, the preparation of which is disclosed in the co-pending European patent applications EP—A—143333 and EP—A—142740. Compounds of the formula (II), in which $R^3$ stands for a hydrogen atom, may be prepared by reacting the corresponding 1-methyl-3,4-dihydroisoquinoline derivatives or the corresponding 1-(B-hydroxyethyl)-3,4-dihydroisoquinoline derivatives with formaldehyde or the hydrate or trimeric derivatives thereof, in an alkalkine medium, and subsequently hydrogenating the product obtained. The compounds in which $R^3$ is other than hydrogen may be prepared from the N-unsubstituted compounds by reactants suitable for the introduction of the desired $R^3$ groups, e.g. alkylation, aralkylation or acylation. The 1-methyl- and 1-(β-hydroxethyl)-3,4-dihydroisoquinoline derivatives used for the preparation of the starting compounds of formula (II) are known in the art and can, for example, be prepared from homoveratryl amine or the corresponding phenylethyl amine derivatives by acylation and conventional isoquinoline cyclization reactions, e.g. Bischler-Napieralski synthesis.

The N-substituted [bis(hydroxymethyl)-methyl]-isoquinoline derivatives of the formular (III) (process b)) are a sub-group of the compounds of formula (II), and can be prepared following the methods used for the preparation of the latter compounds, starting from the corresponding N-unsubstituted compounds.

According to variant $a_1$) the compounds of the formula (I), in which $R^7$ and $R^8$ both stand for a halogen, are prepared by halogenating [bis(hydroxymethyl)-methyl]-isoquinoline derivatives of the formula (II) ($R^1$, $R^2$ and $R^3$ are as hereinbefore defined). The halogenation can be performed with any conventional halogenating agent, under usual conditions of halogenation halides being preferred. Thus chlorination may be carried out with chlorine gas, but the reaction is easier to control if for example sulfinyl dichloride is used as a halogenating agent. Similarly, bromination may be performed with elementary bromine, but it is more preferred to use e.g. phosphorus, tribromide as a brominating agent. The reaction conditions are selected depending the concrete reactants employed.

According to variant $a_2$) compounds of the formula (I), in which $R^7$ and $R^8$ both represent an

$$-O-\underset{\underset{X}{\|}}{C}-R^9 \text{ group,}$$

are prepared by reacting a corresponding compound of the formula (II) with a reactant suitable for introducing an acyl group of the above formula. Depending on the meaning of $R^9$, acylation may be performed with an acid halide or acid anhydride or — if $R^9$ is an —NH—$C_{1-4}$-alkyl or —NH—phenyl group — with a corresponding isocyanate or isothiocyanate, under reaction conditions conventional for acylation reactions.

The N-substituted [bis(hydroxymethyl)-methyl]-isoquinoline derivatives of the formula (III) ($R^1$, $R^2$, $R^4$ and X are as defined above) can be converted into the corresponding O-acyl derivatives by N→O acyl migration, with acid catalysis, preferably as an acid a mineral acid, particularly hydrochloric acid being used N→O acyl migration is a reversible chemical reaction well known in the organic chemistry, which proceeds into O→N direction under alkaline conditions. [G. Bernáth, K. Kovács, K. L. Láng: Acta Chim. Sci. Hung. *65,* 347 (1970)].

Compounds of the formula (I) can be converted into their acid addition salts by reaction with suitable acids.

Salt formation can be carried out, for example, in an inert organic solvent, such as a $C_{1-6}$ aliphatic alcohol, by dissolving the compound of the formula (I) in the solvent and adding the selected acid or a solution thereof formed with the same solvent to the first solution until it becomes slightly acidic (pH 5 to 6). Thereafter the acid addition salt separates and can be removed from the reaction mixture e.g. by filtration.

The compounds of the formula (I) or the salts thereof, if desired, can be subjected to further purification, e.g. recrystallization. The solvents used for recrystallization are selected depending on the solubility and crystallization properties of the compound to be crystallized.

Moreover according to the invention pharmaceutical compositions which contain one or more compound(s) according to the invention as [an] active ingredient(s), suitably together with one or more pharmaceutical carrier(s) and/or excipient(s), are provided for.

As already mentioned before the compounds according to the invention are biologically active, e.g. possess valuable immunosuppressive, antidepressive, analgesic, antipyretic, antihypoxial, gastric acid secretion inhibiting, anticonvulsive and antitetrabenazine activity.

The new compounds of the formula (I) and their physiologically acceptable salts may be formulated for therapeutic purposes. Carriers conventional for this purpose and suitable for parenteral or enteral administration as well as other additives may be used. As carriers solid or liquid compounds, for example water, gelatine, lactose, milk sugar, starch, pectin, magnesium stearate, stearic acid, talc, vegetable oils, such as peanut oil, olive oil, arabic gum, polyalkylene glycols and Vaseline (registered Trade Mark), can be used. The compounds can be formulated as conventional pharmaceutical formulations, for example in a solid (globular and angular pills, dragées, capsules, e.g. hard gelatine capsules) or liquid (injectable oily or aqueous solutions or suspensions) form. The quantity of the solid carrier can be varied within wide ranges, but preferably is between 25 mg and 1 g. The compositions optionally contain also conventional pharmaceutical additives, such as preserving agents, wetting agents, salts for adjusting the osmotic pressure, buffers, flavouring and aroma substances.

The compositions according to the invention optionally contain the compounds of formula (I) in association with other known active ingredients. The unit doses are selected depending on the route of administration. The pharmaceutical compositions are prepared by conventional techniques including sieving, mixing, granulation, pressing or dissolution of the active ingredients. The formulations obtained are then subjected to additional conventional treatment, such as sterilization.

For the pharmacological tests CFLP (LATI) mice of both sexes, weighing 18 to 22 g each and male Han. Wistar (LATI) rats weighing 160 to 180 g each were used. The test material were administered orally, in 30 mg/kg doses, in the form of a suspension containing 5% of Tween® 80, one hour before the tests.

*Test methods*

1. Maximum electroshock (mice)

The shock was applied through a corneal electrode (20mA, 0.2 msec, HSE Schockgerät typ. 207). The animals which do not show a tonic, extensoric spasm as a result of electroshock treatment are considered protected (See Swinyard et al.: J. Pharmacol. Exp. Ther. *106,* 319 (1952)).

2. Metrazole® spasm (mice)

After pretreatment, the animals were administered 125 mg/kg of pentylenetetrazole subcutaneously. The animals which did not show a) a clonic, b) a tonic extensoric spasm and which survived the experiment were regarded protected.

Observation time: one hour (Everett L.M. and Richards R.K.: J. Pharmacol. Exp. Ther. *81,* 402/1944/).

3. Inhibition of tetrabenazine catalepsy

The test was carried out on male rats each weighing 160 to 180 g. The test materials were administered intraperitoneally, in a dose of 30 mg/kg, one hour before tetrabenazine administration. The animals, which if their forlegs were placed on a 7 cm high pillar, did not correct their bizarre position within 30 seconds were regarded cataleptic (Delay J. and Denicker P.: Compt. Rend. Congr. Med. Alenistens Neurologists *19,* 497/Luxemb./).

4. Analgesic activity (mice)

One hour after pretreatment, mice were administered 0.4 ml of a 0.6% acetic acid solution intraperitoneally, as a pain stimulus. The frequency of writhing syndrom is registered for 30 minutes. The changes observed as a result of treatment with the test compounds are related to the mean value of the frequency of writhing syndrom in the control group, and the difference is expressed in percentage (Koster R. et. al.: Exp. Ther. *72*:74 /1941/).

5. Antipyretic activity (rats)

Hyperthermia is induced in rats with Brewer's yeast suspension (0.5% of Brewer's yeast, 1% of arabic gum in a volume of 0.3 ml, s.c.). The animals are treated with the test materials 4 hours later, and the tracheal temperature of the animals is registered with an ELAB thermometer (typ. TM—3) for 4 hours. The antipyretic activity is expressed in percentage of the animals which have an at least one centigrade lower temperature than the average of the control group treated with the solvent (Nimegeers C.J.E. et. al.: Arzneimittel Forsch. *25*:15/9/1975/).

The analgesic activity of 1-[1'-(hydroxymethyl)-1'-(benzoyl-oxymethyl)-methyl]-6,7-dimethoxy-1,2,3,4-tetrahydroisoquinoline hydrochloride (compound A) and 1-[1'-(hydroxymethyl)-1'-(m,p,m'-trimethoxybenzoyloxy-methyl)-methyl]-6,7-diethoxy-1,2,3,4-tetrahydroisoquinoline hydrochloride (compound B) is 4-times and 3.7 times, respectively, higher than that of Na-salicylate. The results are set forth in Table 1.

TABLE 1

| Substances | Antispasm max. electroshock | activity metrazole a | b | Antitetrabenazine activity (%) | Analgesic activity (%) | Antipyretic activity (%) |
|---|---|---|---|---|---|---|
| Compound A | 20.0 | — | 20.0 | 35.0[x] | 28.0[x] | 10.0 |
| Compound B | — | — | 20.0 | — | 30.0[x] | 30.0 |
| Na-salicylate | — | — | 20.0 | — | 113.0[x] | 110.0[x] |

— = ineffective
[x] = $ED_{50}$ (mg/kg)p.o.

The compounds according to the invention are further useful intermediates in the preparation of biologically active 2H-azeto[2,1-a]-isoquinoline derivatives, which are disclosed in the German "Offenlegungsschrift" 34 39 157. The latter compounds may, for example, be prepared by the ring closure of compounds of the formula (I), in which at least one of $R^7$ and $R^8$ stands for halogen, or a salt thereof.

The invention is elucidated in detail by the aid of the following non-limiting Examples.

Example 1

Preparation of 1-[bis(chlormethyl)-methyl]-2-benzoyl-6,7-dimethoxy-1,2,3,4-tetrahydroisoquinoline.

To 0.01 mole (3.7 g) of 1-[bis(hydroxymethyl)-methyl]-2-benzoyl-6,7-dimethoxy-1,2,3,4-tetrahydroisoquinoline 0.04 mole (5 ml) of sulfinyl dichloride is added under ice cooling in about 5 to 10 minutes. The reaction mixture is allowed to stand at room temperature for 24 hours, whereupon the excess of sulfinyl chloride is evaporated, the residue is cooled, alkalized with sodium bicarbonate solution, and extracted with three 30 ml portions of ether. The extract is dried over sodium sulfate and evaporated to about 10 ml. Upon cooling the desired compound is obtained in a crystalline form.

Melting point: 170 to 171°C (ethanol).
Yield: 53%
Analysis for $C_{21}H_{23}Cl_2NO_3$ (408.31):
Calculated: C 61.77%, H 5.68%, N 3.43%, Cl 17.39%;
Found: C 61.35%, H 5.95%, N 3.80%, Cl 17.76%.

The 1-[bis(hydroxymethyl)-methyl]-6,7-dimethoxy-1,2,3,4-tetrahydroisoquinoline used above as a starting material has been prepared as follows.

1-[bis(Hydroxymethyl)-methyl]-2-benzoyl-6,7-dimethoxy-1,2,3,4-tetrahydroisoquinoline 0.01 mole (2.7 g) of 1-[bis(hydroxymethyl)-methyl]-6,7-dimethoxy-1,2,3,4-tetrahydroisoquinoline is dissolved in 50 ml of benzene with slight warming, and a solution of 0.015 mole (0.6 g) of sodium hydroxide in 10 ml of water is added to the solution. 0.011 mole (0.5 g) of benzoyl chloride is added dropwise to the reaction mixture, under cooling and stirring. When the addition of acid chloride is complete, the mixture is stirred at room temperature for one hour, whereupon the organic phase is separated, and the aqueous phase is extracted twice with chloroform. The combined organic phases are dried and evaporated to yield the desired compound.

Melting point: 161 to 163°C (methanol/ether).
Yield: 95%.
Analysis for $C_{21}H_{25}NO_5$ (371.43):
calculated: C 67.91%, H 6.78%, N 3.77%;
found: C 67.72%, H 6.98%, N 4.00%.

Example 2

Preparation of 1-[1'-(hydroxymethyl)-1'-(benzoyloxymethyl)-methyl]-6,7-dimethoxy-1,2,3,4-tetrahydroisoquinoline hydrochloride

0.01 mole (3.7 g) of 1-[bis(hydroxymethyl)-methyl]-2-benzoyl-6,7-dimethoxy-1,2,3,4-tetrahydroisoquinoline prepared as indicated above in the preparation of the identical starting material of Example 1 is dissolved in 20 ml of absolute ethanol. An excess amount of an absolute ethanolic hydrogen chloride solution is added to the solution, and the reaction mixture is then refluxed for 4 hours. Thereafter the reaction mixture is evaporated, and the obtained crystalline product is recrystallized from absolute ethanol.

Melting point: 211 to 214°C.
Yield: 95%.
Analysis for $C_{21}H_{26}ClNO_5$ (407.90):
calculated: C 61.84%, H 6.42%, N 3.43%;
found: C 61.78%, H 6.55%, N 3.60%.

The compounds of formula I/1 set forth in Table 1 can be prepared in an analogous manner, by proper selection of the starting substances. The reflux time, depending on the substituent of the acyl group, is between one and six hours.

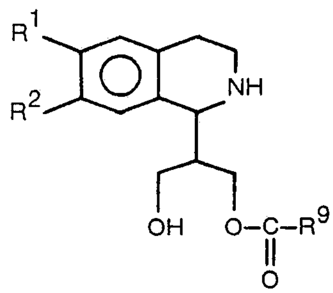

(I/1)

TABLE 1
1-[1'-(Hydroxymethyl)-1'-(acyloxymethyl)-methyl]-6,7-dialkoxy-1,2,3,4-tetrahydroisoquinoline hydrochlorides of formula (I/1)

| Example | $R^1=R^2$ | $R^9$ | Formula/ Molecular weight | Melting point (°C) Solvent | Analysis calcu-lated | found | Yield (%) |
|---|---|---|---|---|---|---|---|
| 3 | $CH_3O$ | $CH_3$ | $C_{16}H_{24}ClNO_5$ | 208—210 | C: 55.57 | 55.97 | |
| | | | 345.82 | ethanol | H: 7.00 | 7.37 | 93 |
| | | | | | N: 4.05 | 4.20 | |
| 4 | $C_2H_5O$ | $CH_3$ | $C_{18}H_{28}ClNO_5$ | 193—197 | C: 57.82 | 58.07 | |
| | | | 373.86 | ethanol | H: 7.55 | 7.92 | 91 |
| | | | | | N: 3.75 | 3.54 | |
| 5 | $C_2H_5O$ | $C_6H_5$ | $C_{23}H_{30}ClNO_2$ | 182—184 | C: 63.37 | 62.96 | |
| | | | 435.95 | ethanol/ | H: 6.94 | 7.20 | 87 |
| | | | | ether | N: 3.21 | 3.39 | |
| 6 | $CH_3O$ | $C_6H_4CH_3(p)$ | $C_{22}H_{28}ClNO_5$ | 221—222 | C: 62.62 | 62.37 | |
| | | | 421.95 | abs. ethanol | H: 6.69 | 7.06 | 82 |
| | | | | | N: 3.32 | 3.41 | |
| 7 | $C_2H_5O$ | $C_6H_4CH_3(p)$ | $C_{24}H_{32}ClNO_5$ | 187—189 | C: 64.07 | 63.32 | |
| | | | 449.95 | EtOH | H: 7.17 | 7.55 | 85 |
| | | | | ether | N: 3.11 | 2.57 | |
| 8 | $CH_3O$ | $C_6H_4Cl(p)$ | $C_{21}H_{25}Cl_2NO_5$ | 220—223 | C: 57.02 | 56.80 | |
| | | | 442.34 | methanol | H: 5.70 | 5.93 | 95 |
| | | | | | N: 3.17 | 3.25 | |

TABLE 1 (continued)

| Example | $R^1=R^2$ | $R^9$ | Formula/ Molecular weight | Melting point (°C) Solvent | Analysis calcu- lated | found | Yield (%) |
|---|---|---|---|---|---|---|---|
| 9 | $C_2H_5O$ | $C_6H_4Cl(p)$ | $C_{23}H_{29}Cl_2NO_5$ 470.40 | 189—192 ethanol | C: 58.73 H: 6.21 N: 2.98 | 58.79 5.99 2.95 | 90 |
| 10 | $C_2H_5O$ | $C_6H_4NO_2(p)$ | $C_{23}H_{29}ClN_2O_7$ 481.95 | 195—198 ethanol/ ether | C: 57.32 H: 6.06 N: 5.81 | 57.13 6.38 5.52 | 85 |
| 11 | $CH_3O$ | $C_6H_3(OCH_3)_2$ (*m, p*) | $C_{23}H_{30}ClNO_7$ 467.94 | 148—150 ethanol | C: 59.03 H: 6.46 N: 2.99 | 58.48 6.07 3.21 | 78 |
| 12 | $C_2H_5O$ | $C_6H_3(OCH_3)_2$ (*m, p*) | $C_{25}H_{34}ClNO_7$ 495.98 | 127—131 ethanol | C: 60.54 H: 6.91 N: 2.82 | 60.12 7.23 2.85 | 78 |
| 13 | $CH_3O$ | $C_6H_2(OCH_3)_3$ (*m, p, m*) | $C_{24}H_{32}ClNO_8$ 497.97 | 160—162 ethanol | C: 57.88 H: 6.48 N: 2.81 | 57.70 6.96 3.17 | 75 |
| 14 | $C_2H_5O$ | $C_6H_2(OCH_3)_3$ (*m, p, m*) | $C_{26}H_{36}ClNO_8$ 526.03 | 155—162 ethanol | C: 59.37 H: 6.90 N: 2.66 | 59.53 7.21 2.86 | 78 |
| 15 | $CH_3O$ | $C_6H_3Cl_2$ (*m, p*) | $C_{21}H_{24}Cl_3NO_5$ 476.78 | 173—176 ethanol | C: 52.90 H: 5.07 N: 2.94 | 52.05 5.39 2.90 | 79 |
| 16 | $C_2H_5O$ | $C_6H_3Cl_2$ (*m, p*) | $C_{23}H_{28}Cl_3NO_5$ 504.83 | 135—140 ethanol | C: 54.72 H: 5.59 N: 2.77 | 54.52 5.52 2.57 | 89 |

Example 17

Preparation of 1-[bis(chloromethyl)-methyl]-6,7-dimethoxy-1,2,3,4-tetrahydroisoquinoline hydrochloride

0.01 mole (3.0 g) of 1-[bis(hydroxymethyl)-methyl]-6,7-dimethoxy-1,2,3,4-tetrahydroisoquinoline hydrochloride is powdered and suspended in 6.5 ml of sulfinyl dichloride. The mixture is then slightly heated under stirring for 30 minutes. The excess of sulfinyl dichloride is distilled off under vacuum (ejector jet pump), the residue is taken up in benzene and evaporated to dryness. The obtained oily residue is crystallized from a mixture of ethanol and ether.

Melting point: 216 to 219°C.
Yield: 76%.
Analysis for $C_{14}H_{20}Cl_3NO_3$ (340.68):

| | | |
|---|---|---|
| calculated: | C 49.36%, | H 5.92%; |
| found: | C 49.76%, | H 6.12%. |

The corresponding diethoxy analogue can be prepared from 1-[bis(hydroxymethyl)-methyl]-6,7-diethoxy-1,2,3,4-tetrahydroisoquinoline hydrochloride in an analogous manner.

Melting point: 149 to 153°C (ethanol/ether).
Yield: 73%.
Analysis for $C_{16}H_{25}Cl_3NO_2$ (368.73):

| | | |
|---|---|---|
| calculated: | C 52.12%, | H 6.56%; |
| found: | C 52.65%, | H 6.37%. |

Example 18

Preparation of 1-[bis(bromomethyl)-methyl]-6,7-diethoxy-1,2,3,4-tetrahydroisoquinoline hydrobromide

To a suspension of 0.01 mole (3.5 g) 1-[bis-(hydroxymethyl)-methyl]-6,7-dimethoxy-1,2,3,4-tetrahydroisoquinoline in 30 ml of absolute benzene 0.03 mole (20 ml) of phosphorus tribromide is portionwise added, under ice cooling and stirring. The reaction mixture is refluxed for 45 minutes. After cooling 20 ml of cold water are added, and the mixture is refluxed for additional 10 minutes. After cooling, the two phases are separated, and the benzene phase is extracted with two 20 ml portions of water. The combined aqueous phases are washed with two 20 ml portions of diethyl ether. The aqueous phase is then adjusted to pH 8 with sodium hydroxide and extracted with four 30 ml portions of ether. The extract is dried and evaporated. A solution of hydrogen bromide in absolute ethanol is added to the residue to yield 1-[bis(bromomethyl)-methyl]-6,7-diethoxy-1,2,3,4-tetrahydroisoquinoline hydrobromide in a crystalline form.

Melting point: 188 to 190°C (ethanol/ether).
Yield: 78%.
Analysis for $C_{16}H_{24}Br_3NO_2$ (502.09):

| | | | |
|---|---|---|---|
| calculated: | C 38.27%, | H 4.82%, | N 2.79%; |
| found: | C 38.26%, | H 4.92%, | N 2.87%. |

Example 19

Preparation of 1-[bis(bromomethyl)-methyl]-6,7-diethoxy-1,2,3,4-tetrahydroisoquinoline hydrobromide

To 0.01 mole (2.7 g) of 1-[bis(hydroxymethyl)-methyl]-6,7-diethoxy-1,2,3,4-tetrahydroisoquinoline 100 ml of absolute tetrachloromethane are added, and the solution is saturated with hydrogen bromide gas under ice cooling. The solvent is evaporated under reduced pressure to yield the hydrobromide of the starting substance as a viscous mass. To this residue 0.03 mole (20 ml) of phosphorus tribromide is added, and the mixture is heated on a water bath for 4 hours. After cooling, the aimed compound is separated from the reaction mixture as a crystalline substance. The compound is filtered off and thoroughly washed with absolute ether or absolute acetone. The physical and spectroscopical data of the compound obtained are identical with those of the compound prepared according to Example 18.

Example 20

Preparation of 1-[1′-(chloromethyl)-1′-(benzoyloxymethyl)-methyl]-6,7-dimethoxy-1,2,3,4-tetrahydroisoquinoline hydrochloride

Starting from 1-[1′-(hydroxymethyl)-1′-(benzoyloxymethyl)-methyl]-6,7-dimethoxy-1,2,3,4-tetrahydroisoquinoline prepared according to Example 2, otherwise following the procedure described in Example 1, the desired compound is obtained with a melting point (after crystallization from 96% ethanol) of 196 to 198°C.

Analysis for $C_{21}H_{25}Cl_2NO$ (426.25):

| | | | | |
|---|---|---|---|---|
| calculated: | C 59.14%, | H 5.91%, | N 3.25%, | Cl 16.63%; |
| found: | C 60.54%, | H 6.20%, | N 3.44%, | Cl 16.58%. |

Example 21

Preparation of 1-[1′-(chloromethyl)-1′-(hydroxymethyl)-methyl]-6,7-dimethoxy-1,2,3,4-tetrahydroisoquinoline hydrochloride

0.01 mole (4.26 g) of 1-[1′-(chloromethyl)-1′-(benzoyloxymethyl)-methyl]-6,7-dimethoxy-1,2,3,4-tetrahydroisoquinoline hydrochloride prepared according to Example 20 is boiled in a mixture of 50 ml of a 10% aqueous hydrogen chloride solution and 10 ml of ethanol for 2.5 hours. After evaporation the desired compound is obtained in a crystalline form.

Melting point (after recrystallization from 96% ethanol): 216—219°C.
Yield: 67%.
Analysis for $C_{14}H_{21}Cl_2NO_3$ (322.23):
calculated: C 52.18%, H 6.56%, N 4.34%, Cl 22.00%;
found: C 52.68%, H 6.56%, N 4.48%, Cl 21.86%.

## Example 22

Preparation of 1-[bis(phenylcarbamoyloxymethyl)-methyl]-2-benzyl-6,7-dimethoxy-1,2,3,4-tetrahydroisoquinoline

A) 1-[bis(Hydroxymethyl)-methyl]-2-benzyl-6,7-dimethoxy-1,2,3,4-tetrahydroisoquinoline hydrochloride

0.01 mole (3.7 g) of the N-benzoyl compound used as a starting material in Example 1 is reacted with 3 g of lithium tetrahydroaluminate (III) in absolute tetrahydrofuran. The reaction mixture is refluxed for 3 hours, worked up in a usual manner, and is then converted into the desired hydrochloride salt.
Melting point: 177 to 179°C (ethanol/ether).
Yield: 69%.
Analysis for $C_{21}H_{28}ClNO_4$ (393.90):
calculated: C 64.03%, H 7.16%, N 3.56%;
found: C 63.65%, H 7.53%, N 3.12%.

B) 0.01 mole of the compound obtained in Step A) is boiled with 0.02 mole of phenyl isocyanate for 4 to 6 hours. Evaporation of the reaction mixture yields the desired compound in a crystalline form.
Melting point: 185°C (toluene).
Analysis for $C_{35}H_{37}N_3O_6$ (595.70):
calculated: C 70.57%, H 6.26%, N 7.05%;
found: C 70.09%, H 6.33%, N 6.54%.

The compounds of formula (I/2) set forth in Table 2 can be prepared in an analogous manner, by proper selection of the starting substances.

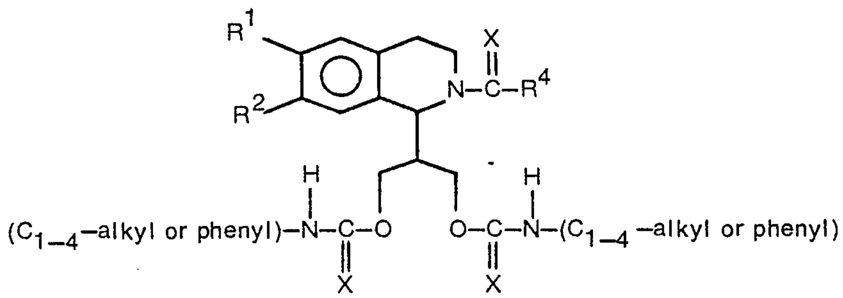

(I/2)

TABLE 2
Compounds of formula (I/2)

| Example | $R^1=R^2$ | $R^3$ | $R^9$ | Formula Molecular weight | Melting point (°C) solvent | Analysis calculated found C | H | N |
|---|---|---|---|---|---|---|---|---|
| 23 | $CH_3O$ | $COC_3H_5$ | $NHC_4H_9$ | $C_{31}H_{43}N_3O_7$ | 87 benzene | 65.36 | 7.61 | 7.37 |
| | | | | 569.70 | | 65.30 | 7.77 | 6.98 |
| 24 | $CH_3O$ | $COC_6H_5$ | $NHC_6H_5$ | $C_{35}H_{35}N_3O_2$ | 182—184 | 68.94 | 5.79 | 6.89 |
| | | | | 609.68 | benzene | 69.81 | 6.17 | 6.97 |
| 25 | $CH_3O$ | $COCH_3$ | $NHC_6H_5$ | $C_{30}H_{33}N_3O_7$ | 164—165 | 65.80 | 6.07 | 7.67 |
| | | | | 547.61 | benzene | 65.44 | 7.07 | 8.28 |
| 26 | $CH_3O$ | $CONHC_6H_5$ | $NHC_6H_5$ | $C_{35}H_{36}N_4O_7$ | 173—174 | 67.29 | 5.81 | 8.97 |
| | | | | 624.70 | benzene | 69.48 | 5.79 | 9.25 |

Example 27

Preparation of 1-[bis(phenylcarbamoyloxymethyl)-methyl]-2-(phenylcarbamoyl)-6,7-dimethoxy-1,2,3,4-tetrahydroisoquinoline

0.01 mole of 1-[bis(hydroxymethyl)-methyl]-6,7-dimethoxy-1,2,3,4-tetrahydroisoquinoline is reacted with 0.03 mole of phenylisocyanate in toluene, as described in Example 22. The desired compound is obtained, which has the same physical and analytical characteristics as the product of Example 26 (Table 2).

Example 28

Preparation of 1-[1′-(hydroxymethyl)-1′-(benzoyloxymethyl)-methyl]-2-benzoyl-6,7-dimethoxy-1,2,3,4-tetrahydroisoquinoline

0.01 mole (4.08 g) of 1-[1′-(hydroxymethyl)-1′-(benzoyloxymethyl)-methyl]-6,7-dimethoxy-1,2,3,4-tetrahydroisoquinoline hydrochloride is suspended in 50 ml of benzene, and to the suspension 25 ml of a 2M aqueous sodium hydroxide solution are added. 0.011 mole of benzoyl chloride is added with stirring to ther reaction mixture and the mixture is stirred at room temperature for one hour. The organic phase is separated and the aqueous phase is extracted with two 50 ml portions of benzene. Drying and evaporation of the combined organic phases yields the desired compound in a crystalline form.

Melting point: 135 to 138°C (benzene).
Yield: 85%.
Analysis for $C_{28}H_{29}NO_6$ (475.56):
calculated: C 70.72%, H 6.15%, N 2.95%;
found: C 71.05%, H 5.92%, N 3.01%.

Example 29

Preparation of 1-[1′-(hydroxymethyl)-1′-(benzoyloxymethyl)-methyl]-2-(N′-phenylthiocarboxamido)-6,7-dimethoxy-1,2,3,4-tetrahydroisoquinoline

5 mmoles (2.04 g) of 1-[1′-(hydroxymethyl)-1′-(benzoyloxymethyl)-methyl]-6,7-dimethoxy-1,2,3,4-tetrahydroisoquinoline hydrochloride are dissolved in 20 ml of water, to the solution 5 ml of a 10% aqueous sodium hydroxide solution are added and it is then extracted with three 20 ml portions of benzene. The combined benzene phases are dried, whereupon 5 mmoles of phenyl isothiocyanate are added. After 24 hours the precipitated product is filtered off and crystallized from ethanol to yield the desired compound.

Melting point: 155 to 158°C.
Yield: 79%.
Analysis for $C_{28}H_{30}N_2O_5S$ (506.61):
calculated: C 66.38%, H 5.97%, N 5.53%;
found: C 66.58%, H 6.35%, N 5.16%.

**Claims for the Contracting States: BE CH DE FR GB IT LI LU NL SE**

1. [Bis(substituted methyl)-methyl]-isoquinoline derivatives of the formula (I)

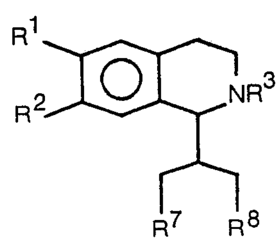

(I),

wherein
$R^1$ and $R^2$ represent hydroxyl or alkoxyl having from 1 to 6 carbon atoms,
$R^3$ is hydrogen, aralkyl having from 1 to 4 carbon atoms in the alkyl moiety or a

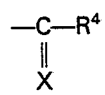

group in which $R^4$ is alkyl having from 1 to 5 carbon atoms or phenyl, optionally substituted by one or more alkyl having from 1 to 4 carbon atoms, alkoxy having from 1 to 4 carbon atoms, halogen and/or nitro, or is —NH-phenyl, X is oxygen or sulfur,
$R^7$ is hydroxyl, halogen or an

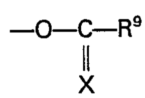

group,

$R^8$ is halogen, alkoxy having from 1 to 4 carbon atoms or an

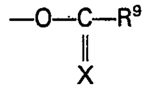

group, in which X has the same meaning as defined above, and $R^9$ independently from $R^4$ may have the same meanings as defined for $R^4$, or is an —NH($C_{1-4}$-alkyl) or $C_{3-6}$-cycloalkyl group, and salts thereof.

2. [Bis(substituted methyl)-methyl]-isoquinoline derivatives as claimed in claim 1, characterized in that the alkyl which may be represented by $R^4$ and/or $R^9$ or constituting the alkyl moiety of the aralkyl which may be represented by $R^3$ or by which the phenyl which may be represented by $R^4$ and/or $R^9$ may be substituted and/or the alkoxy which may be represented by $R^1$ and/or $R^2$ and/or $R^8$ and/or the alkoxy by which the phenyl which may be represented by $R^4$ and/or $R^9$ may be substituted is or are, respectively, such having 1 or 2 carbon atoms and the alkyl of the —NH($C_{1-4}$-alkyl) group which may be represented by $R^9$ is such having 3 or 4 carbon atoms.

3. [Bis(substituted methyl)-methyl]-isoquinoline derivatives as claimed in claim 1 or 2, characterized in that the halogen(s) which may be represented by $R^7$ and/or $R^8$ and/or by which the phenyl which may be represented by $R^4$ and/or $R^9$ may be substituted is or are, respectively, chlorine and/or bromine, particularly chlorine.

4. [Bis(substituted methyl)-methyl]-isoquinoline derivatives as claimed in claim 1 to 3, characterized in that the cycloalkyl which may be represented by $R^9$ is such having 5 or 6, particularly 6 [cyclohexyl], carbon atoms.

5. A process for preparing the compounds according to claims 1 to 4, characterized in reacting a [bis(hydroxymethyl)-methyl]-isoquinoline derivative of the formula (II),

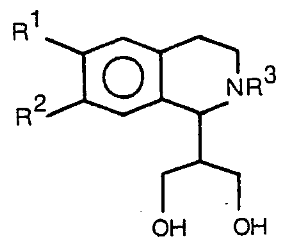

(II),

wherein $R^1$, $R^2$ and $R^3$ are as defined above,

$a_1$) with a halogenating agent to prepare compounds of formula (I), in which $R^7$ and $R^8$ both represent halogen, and, if desired, selectively alkoxylating a compound obtained, to yield compounds of formula (I), in which $R^7$ is halogen and $R^8$ is alkoxy having from 1 to 4 carbon atoms, or

$a_2$) with an acylating agent suitable for the introduction of an

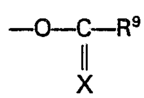

group, to prepare compounds of formula (I), in which $R^7$ and $R^8$ both represent an

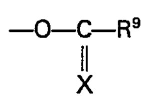

group, in which $R^9$, X, $R^1$, $R^2$ and $R^3$ are as defined above or

b) converting an N-substituted [bis(hydroxymethyl)-methyl]-isoquinoline derivative of the formula (III),

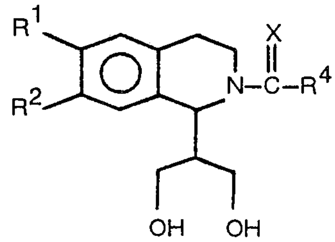

(III),

in which $R^1$, $R^2$, $R^4$ and X are as defined above, into a corresponding O-acyl derivative by N→O acyl

migration, in the presence of a catalytic amount of an acid and, if desired, halogenating a compound of formula (IV) obtained,

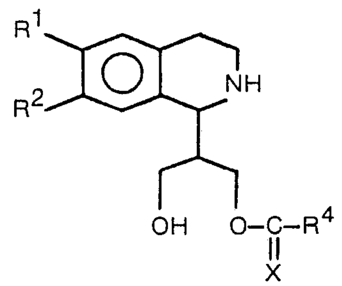

(IV),

in which $R^1$, $R^2$, $R^4$ and X are as defined above, or a salt thereof and, if desired, splitting off the acyl or thioacyl group from a compound of formula (V) obtained,

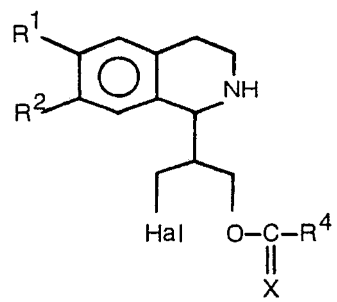

(V),

in which $R^1$, $R^2$, $R^4$ and X are as defined above and Hal represents halogen, or, if desired, converting a compound of formula (V) obtained in which $R^1$, $R^2$, $R^4$ and Hal are as defined above, into a corresponding compound of formula (I), in which $R^3$ represents an

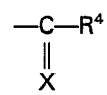

group, by O→N acyl migration, in an alkaline medium and, if desired, from the compounds, in which $R^7$ and/or $R^8$ is acyl, the acyl group(s) can be split off, for example by acid treatment, to yield compounds containing hydroxyl group(s) in the respective position(s), and, if desired, the acyl group(s) can be replaced by other acyl group(s) as well, and/or, if desired, converting a compound of formula (I) obtained into a salt thereof or converting a salt of a compound of formula (I) obtained into the corresponding free base of formula (I) and/or into another salt.

6. A process as claimed in claim 5, characterized in that in process variant $a_1$) halogenation is carried out with a halide.

7. A process as claimed in claim 5, characterized in that in process variant $a_2$) an acid halide or acid anhydride is employed as an acylating agent suitable for the introduction of an

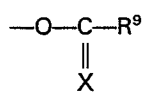

group.

8. A process as claimed in claim 5, characterized in that a corresponding isocyanate or isothiocyanate is used in process variant $a_2$) as an acylating agent suitable for the introduction of an

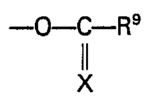

group, in which $R^9$ is an —NH—($C_{1-4}$-alkyl) or —NH-phenyl group.

9. A process as claimed in claim 5, characterized in that in process variant b) a mineral acid, preferably hydrochloric acid, is employed as an acid.

10. Pharmaceutical compositions characterized by a content of one or more compound(s) according to claims 1 to 4 as [an] active ingredient(s), suitably together with one or more pharmaceutical carrier(s) and/or excipient(s).

**Claims for the Contracting State: AT**

1. A process for preparing [bis(substituted methyl)-methyl]-isoquinoline derivatives of the formula (I)

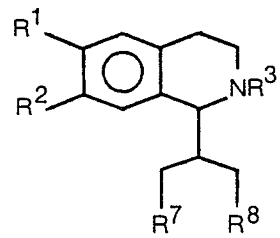

(I),

wherein

$R^1$ and $R^2$ represent hydroxyl or alkoxyl having from 1 to 6 carbon atoms,

$R^3$ is hydrogen, aralkyl having from 1 to 4 carbon atoms in the alkyl moiety or a

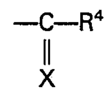

group in which $R^4$ is alkyl having from 1 to 5 carbon atoms or phenyl, optionally substituted by one or more alkyl having from 1 to 4 carbon atoms, alkoxy having from 1 to 4 carbon atoms, halogen and/or nitro, or is —NH-phenyl, X is oxygen or sulfur,

$R^7$ is hydroxyl, halogen or an

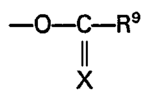 group,

$R^8$ is halogen, alkoxy having from 1 to 4 carbon atoms or an

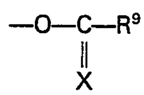

group, in which X has the same meaning as defined above, and $R^9$ independently from $R^4$ may have the same meanings as defined for $R^4$, or is an —NH($C_{1-4}$-alkyl) or $C_{3-6}$-cycloalkyl group,

and salts thereof, characterized in reacting a [bis(hydroxymethyl)-methyl]isoquinoline derivative of the formula (II)

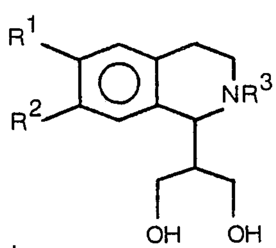

(II),

wherein $R^1$, $R^2$ and $R^3$ are as defined above,

$a_1$) with a halogenating agent to prepare compounds of formula (I), in which $R^7$ and $R^8$ both represent halogen, and, if desired, selectively alkoxylating a compound obtained, to yield compounds of formula (I), in which $R^7$ is halogen and $R^8$ is alkoxy having from 1 to 4 carbon atoms, or

$a_2$) with an acylating agent suitable for the introduction of an

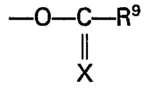

group, to prepare compounds of formula (I), in which $R^7$ and $R^8$ both represent an

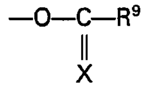

group, in which $R^9$, X, $R^1$, $R^2$ and $R^3$ are as defined above or

b) converting an N-substituted [bis(hydroxymethyl)-methyl]-isoquinoline derivative of the formula (III),

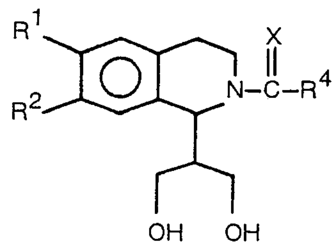

(III),

in which $R^1$, $R^2$, $R^4$ and X are as defined above, into a corresponding O-acyl derivative by N→O acyl migration, in the presence of a catalytic amount of an acid and, if desired, halogenating a compound of formula (IV) obtained,

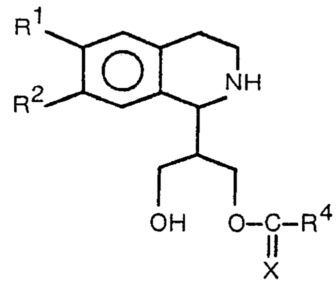

(IV),

in which $R^1$, $R^2$, $R^4$ and X are as defined above, or a salt thereof and, if desired, splitting off the acyl or thioacyl group from a compound of formula (V) obtained,

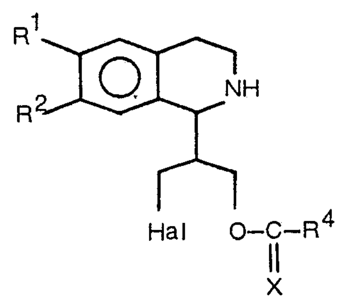

(V),

in which $R^1$, $R^2$, $R^4$ and X are as defined above and Hal represents halogen, or, if desired, converting a compound of formula (V) obtained in which $R^1$, $R^2$, $R^4$ and Hal are as defined above, into a corresponding compound of formula (I), in which $R^3$ represents an

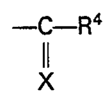

group, by O→N acyl migration, in an alkaline medium and, if desired, from the compounds, in which $R^7$ and/or $R^8$ is acyl, the acyl group(s) can be split off, for example by acid treatment, to yield compounds containing hydroxyl group(s) in the respective position(s), and, if desired, the acyl group(s) can be replaced by other acyl group(s) as well, and/or, if desired, converting a compound of formula (I) obtained into a salt thereof or converting a salt of a compound of formula (I) obtained into the corresponding free base of formula (I) and/or into another salt.

2. A process as claimed in claim 1, characterized in that in process variant $a_1$) halogenation is carried out with a halide.

3. A process as claimed in claim 1, characterized in that in process variant $a_2$) an acid halide or acid anhydride is employed as an acylating agent suitable for the introduction of an

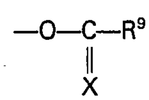

group.

4. A process as claimed in claim 1, characterized in that a corresponding isocyanate or isothiocyanate is used in process variant $a_2$) as an acylating agent suitable for the introduction of an

$$-O-\underset{\underset{X}{\|}}{C}-R^9$$

group, in which $R^9$ is an —NH—($C_{1-4}$-alkyl) or —NH-phenyl group.

5. A process as claimed in claim 1, characterized in that in process variant b) a mineral acid, preferably hydrochloric acid, is employed as an acid.

6. A process according claim 1, characterized in that [bis(substituted methyl)-methyl]-isoquinoline derivatives are prepared in which the alkyl may be represented by $R^4$ and/or $R^9$ or constituting the alkyl moiety of the aralkyl which may be represented by $R^3$ or by which the phenyl which may be represented by $R^4$ and/or $R^9$ may be substituted and/or the alkoxy which may be represented by $R^1$ and/or $R^2$ and/or $R^8$ and/or the alkoxy by which the phenyl which may be represented by $R^4$ and/or $R^9$ may be substituted is or are, respectively, such having 1 or 2 carbon atoms and the alkyl of the —NH($C_{1-4}$-alkyl) group which may be represented by $R^9$ is such having 3 or 4 carbon atoms.

7. A process according to claim 1, characterized in that [bis(substituted methyl)-methyl]-isoquinoline derivatives are prepared in which halogen(s) which may be represented by $R^7$ and/or $R^8$ and/or by which the phenyl which may be represented by $R^4$ and/or $R^9$ may be substituted is or are, respectively, chlorine and/or bromine, particularly chlorine.

8. A process according to claim 1, characterized in that [bis(substituted methyl)-methyl]-isoquinoline derivatives are prepared in which the cycloalkyl which may be represented by $R^9$ is such having 5 or 6, particularly 6 [cyclohexyl], carbon atoms.

**Patentansprüche für die Vertragsstaaten: BE CH DE FR GB IT LI LU NL SE**

1. Gegenstand der Erfindung sind [Bis(substituierte methyl)-methyl]-isochinolinderivate der Formel (I)

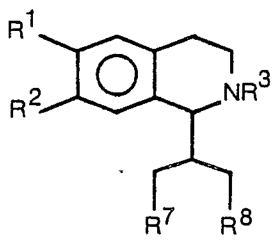

(I),

wobei

$R^1$ und $R^2$ Hydroxyl oder Alkoxyl mit 1 bis 6 Kohlenstoffatomen darstellen,

$R^3$ für Wasserstoff, Aralkyl mit 1 bis 4 Kohlenstoffatomen im Alkylanteil oder eine

$$-\underset{\underset{X}{\|}}{C}-R^4\text{-Gruppe steht, in der}$$

$R^4$ Alkyl mit 1 bis 5 Kohlenstoffatomen oder Phenyl, wahlweise durch ein oder mehrere Alkyle mit 1 bis 4 Kohlenstoffatomen substituiert, Alkoxyl mit 1 bis 4 Kohlenstoffatomen, Halogen und/oder Nitro ist, oder für —NH-Phenyl steht, X Sauerstoff oder Schwefel ist,

$R^7$ Hydroxyl, Halogen oder eine

$$-O-\underset{\underset{X}{\|}}{C}-R^9\text{-Gruppe ist,}$$

$R^8$ für Halogen, Alkoxyl mit 1 bis 4 Kohlenstoffatomen oder eine

$$-O-\underset{\underset{X}{\|}}{C}-R^9\text{-Gruppe steht,}$$

in der X die oben festgelegte Bedeutung hat, und $R^9$ unabhängig von $R^4$ die wie für $R^4$ festgelegte Bedeutung haben kann, oder für eine —NH($C_{1-4}$-Alkyl) oder $C_{3-6}$-Cycloalkylgruppe steht,

und ihre Salze.

2. [Bis(substituierte methyl)-methyl]-isochinolinderivate gemäß Anspruch 1, dadurch gekennzeichnet, daß das Alkyl, das durch $R^4$ und/oder $R^9$ dargestellt werden kann, oder das den Alkylanteil des Aralkyls bildet, das durch $R^3$ dargestellt werden kann, oder durch das das Phenyl, das durch $R^4$ und/oder $R^9$ dargestellt werden kann, substituiert werden kann, und/oder das Alkoxyl, das durch $R^1$ und/oder $R^2$ und/oder $R^8$ dargestellt werden kann, und/oder das Alkoxyl, durch das das Phenyl, das durch $R^4$ und/oder $R^9$

dargestellt werden kann, substituiert werden kann, 1 bis 2 Kohlenstoffatome auf, und das Alkyl der —NH($C_{1-4}$-Alkyl)-Gruppe, die durch $R^9$ dargestellt werden kann, weist 3 bis 4 Kohlenstoffatome auf.

3. [Bis(substituierte methyl)-methyl]-isochinolinderivate gemäß der Anspruch 1 oder 2, dadurch gekennzeichnet, daß das/die Halogen(e), das/die durch $R^7$ und/oder $R^8$ dargestellt werden kann/können und/oder durch das/die das Phenyl, das durch $R^4$ und/oder $R^9$ dargestellt werden kann, substituiert werden kann, Chlor und/oder Brom, insbesondere Chlor ist bzw. sind.

4. [Bis(substituierte methyl)-methyl]-isochinolinderivate gemäß der Anspruch 1 bis 3, dadurch gekennzeichnet, daß das Cycloalkyl, das durch $R^9$ dargestellt werden kann, ein solches ist, das 5 oder 6, insbesondere 6 [Cyclohexyl], Kohlenstoffatome aufweist.

5. Ein Verfahren zur Herstellung der Verbindungen gemäß der Ansprüche 1 bis 4, gekennzeichnet durch die Umsetzung eines [Bis(hydroxymethyl)-methyl]-isochinolinderivats der Formel (II)

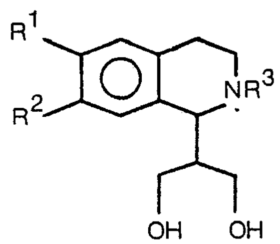

(II),

wobei $R^1$, $R^2$ und $R^3$ wie oben festgelegt sind,

$a_1$) mit einem Halogenisierungsmittel, um Verbindungen der Formel (I) herzustellen, in denen sowohl $R^7$ als auch $R^8$ Halogen darstellen, und ggf. selektive Alkoxylierung einer erhaltenen Verbindung, um Verbindungen der Formel (I) zu liefern, in denen $R^7$ für Halogen und $R^8$ für ein Alkoxyl mit 1 bis 4 Kohlenstoffatomen steht, oder

$a_2$) mit einem zur Einführung einer

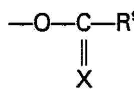-Gruppe

geeigneten Acylierungsmittel, um Verbindungen der Formel (I) herzustellen, in denen $R^7$ und $R^8$ eine

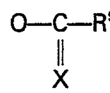-Gruppe

darstellen, in der $R^9$, X, $R^1$, $R^2$ und $R^3$ wie oben festgelegt sind, oder

b) Umwandlung eines N-substituierten [Bis(hydroxymethyl)-methyl]-isochinolinderivats der Formel (III)

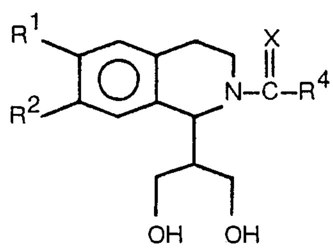

(III),

in der $R^1$, $R^2$, $R^4$ und X wie oben festgelegt sind, in ein entsprechendes O-Acylderivat durch N→O Acylwanderung in Gegenwart eines katalytischen Säureanteils und, ggf., Halogenierung einer erhaltenen Verbindung der Formel (IV),

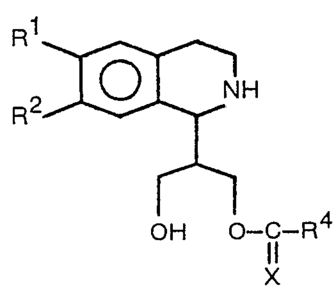

(IV),

in der $R^1$, $R^2$, $R^4$ und X wie oben festgelegt sind, oder ihres Salzes, und, ggf., Abspaltung der Acyl- oder Thioacylgruppe von einer erhaltenen Verbindung der Formel (V)

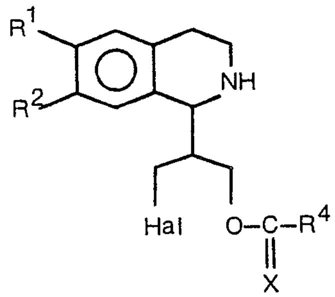

(V),

in der $R^1$, $R^2$, $R^4$ und X wie oben festgelegt sind und Hal Halogen darstellt, oder, ggf., Umwandlung einer erhaltenen Verbindung der Formel (V), in der $R^1$, $R^2$, $R^4$ und Hal wie oben festgelegt sind, in eine entsprechende Verbindung der Formel (I), in der $R^3$ eine

$$-\underset{\underset{X}{\|}}{C}-R^4\text{-Gruppe}$$

darstellt, durch O→N Acylwanderung in einem Alkalimedium, und, ggf., kann von den Verbindungen, in denen $R^7$ und/oder $R^8$ Acyl ist, die Acylgruppe(n) abgespalten werden, z.B. durch Säurebehandlung, um Verbindungen zu liefern, die (eine) Hydroxylgruppe(n) in der/den entsprechenden Stellung(en) enthalten, und, ggf., kann/können die Acylgruppe(n) auch durch (eine) andere Acylgruppe(n) ersetzt werden, und/oder, ggf., Umwandlung einer erhaltenen Verbindung der Formel (I) in ihr Salz oder Umwandlung eines Salzes der erhaltenen Verbindung der Formel (I) in die entsprechende freie Base der Formel (I) und/oder in ein anderes Salz.

6. Ein Verfahren gemäß Anspruch 5, dadurch gekennzeichnet, daß in der Verfahrensvariante $a_1$) die Halogenierung mit einem Halogenid durchgeführt wird.

7. Ein Verfahren gemäß Anspruch 5, dadurch gekennzeichnet, daß in der Verfahrensvariante $a_2$) ein Säurehalogenid oder Säureanhydrid als ein für die Einführung einer

$$-O-\underset{\underset{X}{\|}}{C}-R^9-\text{Gruppe} \quad .$$

geeignetes Acylierungsmittel eingesetzt wird.

8. Ein Verfahren gemäß Anspruch 5, dadurch gekennzeichnet, daß ein entsprechendes Isocyanat oder Isothiocyanat in der Verfahrensvariante $a_2$) als ein Acylierungsmittel verwendet wird, das für die Einführung einer

$$-O-\underset{\underset{X}{\|}}{C}-R^9$$

Gruppe geeignet ist, in der $R^9$ eine —NH—($C_{1-4}$-Alkyl-) oder —NH-Phenylgruppe darstellt.

9. Ein Verfahren gemäß Anspruch 5, dadurch gekennzeichnet, daß in der Verfahrensvariante b) eine Mineralsäure, vorzugsweise Salzsäure, als Säure eingesetzt wird.

10. Arzneimittel, gekennzeichnet durch einen Anteil von einer oder mehreren Verbindung(en) gemäß der Ansprüche 1 bis 4 als (einen) aktive(n) Bestandteil(e), zweckmäßigerweise zusammen mit einem oder mehreren pharmazeutischen Träger(n) und/oder Trägerstoff(en).

**Patentansprüche für den Vertragsstaat: AT**

1. Ein Verfahren zur Herstellung von [Bis(substituierten methyl)-methyl]-isochinolinderivaten der Formel (I)

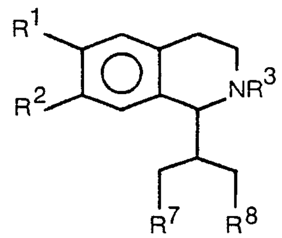

(I),

wobei

$R^1$ und $R^2$ Hydroxyl oder Alkoxyl mit 1 bis 6 Kohlenstoffatomen darstellen,

$R^3$ für Wasserstoff, Aralkyl mit 1 bis 4 Kohlenstoffatomen im Alkylanteil oder eine

—C—$R^4$-Gruppe steht, in der
‖
X

$R^4$ Alkyl mit 1 bis 5 Kohlenstoffatomen oder Phenyl, wahlweise durch ein oder mehrere Alkyle mit 1 bis 4 Kohlenstoffatomen substituiert, Alkoxyl mit 1 bis 4 Kohlenstoffatomen, Halogen und/oder Nitro ist, oder für —NH-Phenyl steht, X Sauerstoff oder Schwefel ist,

$R^7$ Hydroxyl, Halogen oder eine

—O—C—$R^9$-Gruppe ist,
‖
X

$R^8$ für Halogen, Alkoxyl mit 1 bis 4 Kohlenstoffatomen oder eine

—O—C—$R^9$-Gruppe steht,
‖
X

in der X die oben festgelegte Bedeutung hat, und $R^9$ unabhängig von $R^4$ die wie für $R^4$ festgelegte Bedeutung haben kann, oder für eine —NH($C_{1-4}$-Alkyl-) oder $C_{3-6}$-Cycloalkylgruppe steht,

und ihre Salze, gekennzeichnet durch die Umsetzung eines [Bis(hydroxymethyl)-methyl]-isochinoderivats der Formel (II)

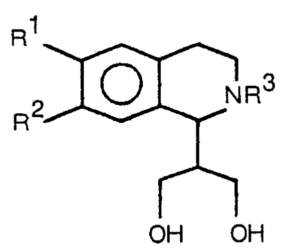

(II),

wobei $R^1$, $R^2$ und $R^3$ wie oben festgelegt sind,

$a_1$) mit einem Halogenisierungsmittel, um Verbindungen der Formel (I) herzustellen, in denen sowohl $R^7$ als auch $R^8$ Halogen darstellen, und ggf. selektive Alkoxylierung einer erhaltenen Verbindung, um Verbindungen der Formel (I) zu liefern, in denen $R^7$ für Halogen und $R^8$ für ein Alkoxyl mit 1 bis 4 Kohlenstoffatomen steht, oder

$a_2$) mit einem zur Einführung einer

—O—C—$R^9$-Gruppe
‖
X

geeigneten Acylierungsmittel, um Verbindungen der Formel (I) herzustellen, in denen $R^7$ und $R^8$ eine

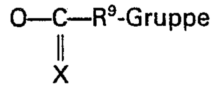

darstellen, in der $R^9$, X, $R^1$, $R^2$ und $R^3$ wie oben festgelegt sind, oder

b) Umwandlung eines N-substituierten [Bis(hydroxymethyl)-methyl]-isochinolinderivats der Formel (III)

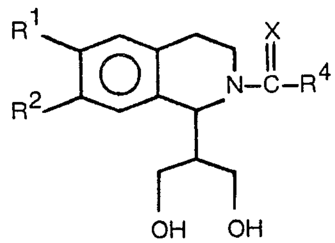

(III),

in der $R^1$, $R^2$, $R^4$ und X wie oben festgelegt sind, in ein entsprechendes O-Acylderivat durch N→O Acylwanderung in Gegenwart eines katalytischen Säureanteils und, ggf., Halogenierung einer erhaltenen Verbindung der Formel (IV),

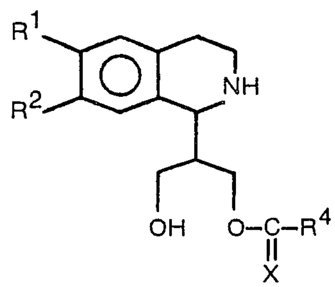

(IV),

in der $R^1$, $R^2$, $R^4$ und X wie oben festgelegt sind, oder ihres Salzes, und, ggf., Abspaltung der Acyl- oder Thioacylgruppe von einer erhaltenen Verbindung der Formel (V)

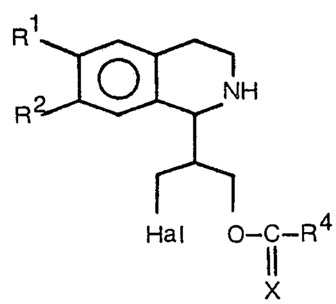

(V),

in der $R^1$, $R^2$, $R^4$ und X wie oben festgelegt sind und Hal Halogen darstellt, oder, ggf., Umwandlung einer erhaltenen Verbindung der Formel (V), in der $R^1$, $R^2$, $R^4$ und Hal wie oben festgelegt sind, in eine entsprechende Verbindung der Formel (I), in der $R^3$ eine

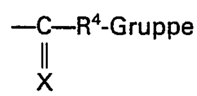

darstellt, durch O→N Acylwanderung in einem Alkalimedium, und, ggf., kann von den Verbindungen, in denen $R^7$ und/oder $R^8$ Acyl ist, die Acylgruppe(n) abgespalten werden, z.B. durch Säurebehandlung, um Verbindungen zu liefern, die (eine) Hydroxylgruppe(n) in der/den entsprechenden Stellung(en) enthalten, und, ggf., kann/können die Acylgruppe(n) auch durch (eine) andere Acylgruppe(n) ersetzt werden, und/oder, ggf., Umwandlung einer erhaltenen Verbindung der Formel (I) in ihr Salz oder Umwandlung eines Salzes der erhaltenen Verbindung der Formel (I) in die entsprechende freie Base der Formel (I) und/oder in ein anderes Salz.

2. Ein Verfahren gemäß Anspruch 1, dadurch gekennzeichnet, daß in der Verfahrensvariante $a_1$) die Halogenierung mit einem Halogenid durchgeführt wird.

3. Ein Verfahren gemäß Anspruch 1, dadurch gekennzeichnet, daß in der Verfahrensvariante $a_2$) ein Säurehalogenid oder Säureanhydrid als ein für die Einführung einer

—O—C—$R^9$—Gruppe
‖
X

geeignetes Acylierungsmittel eingesetzt wird.

4. Ein Verfahren gemäß Anspruch 1, dadurch gekennzeichnet, daß ein entsprechendes Isocyanat oder Isothiocyanat in der Verfahrensvariante $a_2$) als ein Acylierungsmittel verwendet wird, das für die Einführung einer

—O—C—$R^9$
‖
X

Gruppe geeignet ist, in der $R^9$ eine —NH—($C_{1-4}$-Alkyl-) oder —NH-Phenylgruppe darstellt.

5. Ein Verfahren gemäß Anspruch 1, dadurch gekennzeichnet, daß in der Verfahrensvariante b) eine Mineralsäure, vorzugsweise Salzsäure, als Säure eingesetzt wird.

6. Ein Verfahren gemäß Anspruch 1, dadurch gekennzeichnet, daß [Bis(substituierte methyl)-methyl]-isochinolinderivate hergestellt werden, in denen das Alkyl, das durch $R^4$ und/oder $R^9$ dargestellt werden kann, oder das den Alkylanteil des Aralkyls bildet, das durch $R^3$ dargestellt werden kann, oder durch das das Phenyl, das durch $R^4$ und/oder $R^9$ dargestellt werden kann, substituiert werden kann, und/oder das Alkoxyl, das durch $R^1$ und/oder $R^2$ und/oder $R^8$ dargestellt werden kann, und/oder das Alkoxyl, durch das das Phenyl, das durch $R^4$ und/oder $R^9$ dargestellt werden kann, substituiert werden kann, 1 bis 2 Kohlenstoffatome auf, und das Alkyl der —NH($C_{1-4}$-Alkyl)-Gruppe, die durch $R^9$ dargestellt werden kann, weist 3 bis 4 Kohlenstoffatome auf.

7. Ein Verfahren gemäß Anspruch 1, dadurch gekennzeichnet, daß [Bis(substituierte methyl)-methyl]-isochinolinderivate hergestellt werden, in denen das/die Halogen(e), das/die durch $R^7$ und/oder $R^8$ dargestellt werden kann/können und/oder durch das/die das Phenyl, das durch $R^4$ und/oder $R^9$ dargestellt werden kann, substituiert werden kann, Chlor und/oder Brom, insbesondere Chlor ist bzw. sind.

8. Ein Verfahren gemäß Anspruch 1, dadurch gekennzeichnet, daß [Bis(substituierte methyl)-methyl]-isochinolinderivate hergestellt werden, in denen das Cycloalkyl das durch $R^9$ dargestellt werden kann, ein solches ist, das 5 oder 6, insbesondere 6 [Cyclohexyl], Kohlenstoffatome aufweist.

**Revendications pour les Etats contractants: BE CH DE FR GB IT LI LU NL SE**

1. Dérivés de [bis(méthyl-substitué)méthyl]-isoquinoléine de formule (I):

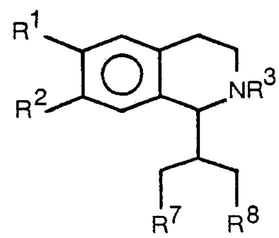

(I),

dans laquelle

$R^1$ et $R^2$ représentent un groupe hydroxy ou alcoxy ayant 1 à 6 atomes de carbone,

$R^3$ est un atome d'hydrogène, ou groupe aralcoyle ayant 1 à 4 atomes de carbone dans la portion alcoyle ou un groupe

—C—$R^4$
‖
X

dans lequel $R^4$ est un groupe alcoyle ayant 1 à 5 atomes de carbone ou un groupe phényle, éventuellement substitué par un ou plusieurs groupes alcoyles ayant 1 à 4 atomes de carbone, alcoxy ayant 1 à 4 atomes de carbone, un atome d'halogène et/ou un groupe nitro, ou il est —NH-phényle, X est un atome d'oxygène ou de soufre,

$R^7$ est un groupe hydroxy, un atome d'halogène ou un groupe

—O—C—$R^9$
‖
X

$R^8$ est un atome d'halogène, un groupe alcoxy ayant 1 à 4 atomes de carbone ou un groupe

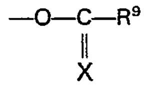

dans lequel X est tel que défini plus haut et $R^9$ indépendamment de $R^4$ peut avoir les même significations que $R^4$, ou il est un groupe —NH(alcoyle en $C_{1-4}$) ou cycloalkyle en $C_{3-6}$ et leurs sels.

2. Dérivés de [bis(méthyl-substitué)méthyl]isoquinoléine suivant la revendication 1, caractérisés en ce que le groupe alcoyle qui peut être représenté par $R^4$ et/ou $R^9$ ou constituant la partie alcoyle du groupe aralcoyle qui peut être représenté par $R^3$ ou par lequel le groupe phényle, qui peut être représenté par $R^4$ et/ou $R^9$ peut être substitué et/ou le groupe alcoxy qui peut être représenté par $R^1$ et/ou $R^2$ et/ou $R^8$ et/ou le groupe alcoxy par lequel le groupe phényle, qui peut être représenté par $R^4$ et/ou $R^9$, peut être substitué, ont respectivement 1 ou 2 atomes de carbone et la partie alcoyle du groupe —NH— (alcoyle en $C_{1-4}$) qui peut être représenté par $R^9$, a 3 ou 4 atomes de carbone.

3. Dérivés de [bis(méthyl-substitué)méthyl]isoquinoléine suivant la revendication 1 ou 2, caractérisés en ce que l'atome ou les atomes d'halogène qui peuvent être représentés par $R^7$ et/ou $R^8$ et/ou par lesquels le groupe phényle, qui peut être représenté par $R^4$ et/ou $R^9$, peut être substitué, sont respectivement des atomes de chlore et/ou de brome, en particulier de chlore.

4. Dérivés de [bis(méthyl-subsitué)méthyl]isoquinoléine suivant les revendications 1 à 3, caractérisés en ce que le groupe cycloalcoyle qui peut être représenté par $R^9$, a 5 ou 6, en particulier 6 atomes de carbone (cyclohexyle).

5. Procédé pour préparer les composés suivant les revendications 1 à 4, caractérisé en ce que:

l'on fait réagir un dérivé de [bis(hydroxyméthyl)méthyl]isoquinoléine de formule (II)

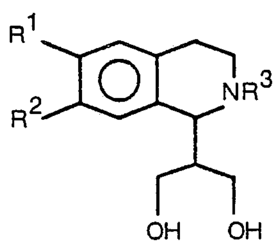

(II),

dans laquelle $R^1$, $R^2$ et $R^3$ sont tels que définis plus haut

$a_1$) avec un agent halogénant pour préparer des composés de formule (I), dans laquelle $R^7$ et $R^8$ représentent tous deux un atome d'halogène, et, si cela est désiré, on soumet à une alcoxylation sélective un composé obtenu, pour fournir des composés de formule (I), dans laquelle $R^7$ est un atome d'halogène et $R^8$ est un groupe alcoxy ayant 1 à 4 atomes de carbone, ou

$a_2$) avec un agent acylant convenable pour introduire un groupe

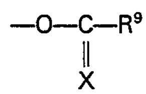

pour préparer des composés de formule (I), dans laquelle $R^7$ et $R^8$ représentent tous deux un groupe

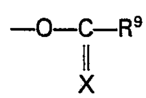

dans lequel $R^9$, X, $R^1$, $R^2$ et $R^3$ sont tels que définis plus haut, ou

b) on convertit un dérivé de [bis(hydroxyméthyl)méthyl]isoquinoléine N-substitué de formule (III):

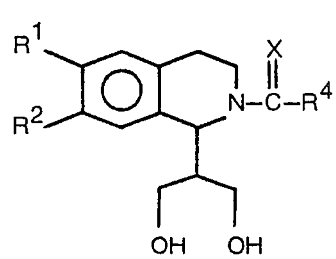

(III),

dans laquelle $R^1$, $R^2$, $R^4$ et X sont tels que définis plus haut, en un dérivé O-acyle correspondant par une migration N→O-acyle, en présence d'une quantité catalytique d'un acide et,

si cela est désiré, on halogène un composé de formule (IV) ainsi obtenu

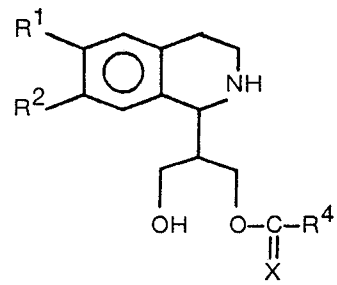

(IV),

dans laquelle $R^1$, $R^2$, $R^4$ et X sont tels que définis plus haut, ou un sel de celui-ci et, si cela est désiré, on coupe le groupe acyle ou thioacyle d'un composé de formule (V):

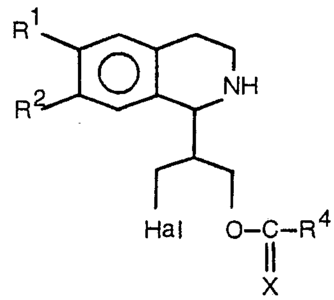

(V),

dans laquelle $R^1$, $R^2$, $R^4$ et X sont tels que définis plus haut et Hal représente un atome d'halogène, ou, si cela est désiré,

on convertit un composé de formule (V) ainsi obtenu dans laquelle $R^1$, $R^2$, $R^4$ et Hal sont tels que définis plus haut, en un composé correspondant de formule (I), dans laquelle $R^3$ représente un groupe

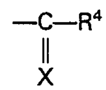

par une migration O→ —N-acyle, dans un milieu alcalin,

et si cela est désiré, on peut détacher à partir des composés dans lesquels $R^7$ et/ou $R^8$ sont des groupes acyles, le ou les groupes acyles par exemple par un traitement à l'acide, pour donner des composés contenant un ou des groupes hydroxy aux positions respectives, et, si cela est désiré, le ou les groupes acyles peuvent être remplacés par d'autres groupes acyles également et/ou, si cela est désiré,

on convertit un composé de formule (I) ainsi obtenu en un sel de celui-ci, ou l'on convertit un sel d'un composé de formule (I) ainsi obtenu en la base libre correspondante de formule (I) et/ou en un autre sel.

6. Procédé suivant la revendication 5, caractérisés en ce que l'halogénation est effectuée avec un halogénure dans la variante $a_1$) du procédé.

7. Procédé suivant la revendication 5, caractérisés en ce que dans la variante $a_2$) du procédé, on emploie un halogénure d'acide ou un anhydride d'acide comme agent acylant convenable pour introduire un groupe

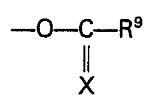

8. Procédé suivant la revendication 5, caractérisé en ce qu'un isocyanate ou un isothiocyanate correspondant est utilisé dans la variante $a_2$) du procédé comme agent acylant convenable pour introduire un groupe

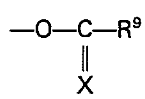

dans lequel $R^9$ est un groupe —NH(alcoyle en $C_{1-4}$) ou NH-phényle.

9. Procédé suivant la revendication 5, caractérisés en ce que dans la variante b) du procédé, un acide minéral, de préférence l'acide chlorhydrique, est utilisé en tant qu'acide.

10. Compositions pharmaceutiques caractérisées en ce qu'elles contiennent un ou plusieurs composés suivant les revendications 1 à 4 en tant que composants actifs convenablement avec un ou plusieurs supports et/ou excipients pharmaceutiques.

**Revendications pour l'Etat contractant: AT**

1. Procédé pour préparer dérivés de [bis(méthyl-substitué)méthyl]-isoquinoléine de formule (I):

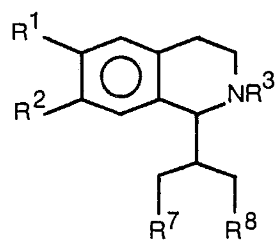

(I),

dans laquelle

$R^1$ et $R^2$ représentent un groupe hydroxy ou alcoxy ayant 1 à 6 atomes de carbone,

$R^3$ est un atome d'hydrogène, un groupe aralcoyle ayant 1 à 4 atomes de carbone dans la portion alcoyle ou un groupe

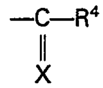

dans lequel $R^4$ est un groupe alcoyle ayant 1 à 5 atomes de carbone ou un groupe phényle, éventuellement substitué par un ou plusieurs groupes alcoyles ayant 1 à 4 atomes de carbone, alcoxy ayant 1 à 4 atomes de carbone, un atome d'halogène et/ou un groupe nitro, ou il est —NH-phényle, X est un atome d'oxygène ou de soufre,

$R^7$ est un groupe hydroxy, un atome d'halogène ou un groupe

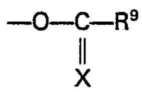

$R^8$ est un atome d'halogène, un groupe alcoxy ayant 1 à 4 atomes de carbone ou un groupe

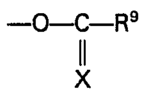

dans lequel X est tel que défini plus haut et $R^9$ indépendamment de $R^4$·peut avoir les même significations que $R^4$, ou il est un groupe —NH(alcoyle en $C_{1-4}$) ou cycloalkyle en $C_{3-6}$ et leurs sels, caractérisé en ce que: l'on fait réagir un dérivé de [bis(hydroxyméthyl)méthyl]-isoquinoléine de formule (II)

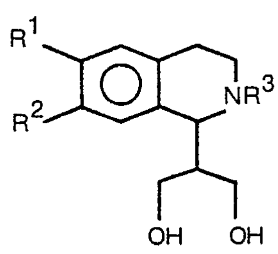

(II),

dans laquelle $R^1$, $R^2$ et $R^3$ sont tels que définis plus haut

$a_1$) avec un agent halogénant pour préparer des composés de formule (I), dans laquelle $R^7$ et $R^8$ représentent tous deux un atome d'halogène, et, si cela est désiré, on soumet à une alcoxylation sélective un composé obtenu, pour fournir des composés de formule (I), dans laquelle $R^7$ est un atome d'halogène et $R^8$ est un groupe alcoxy ayant 1 à 4 atomes de carbone, ou

$a_2$) avec un agent acylant convenable pour introduire un groupe

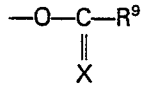

pour préparer des composés de formule (I), dans laquelle $R^7$ et $R^8$ représentent tous deux un groupe

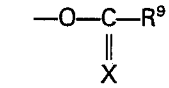

dans lequel $R^9$, X, $R^1$, $R^2$ et $R^3$ sont tels que définis plus haut, ou

b) on convertit un dérivé de [bis(hydroxyméthyl)méthyl]isoquinoléine N-substitué de formule (III):

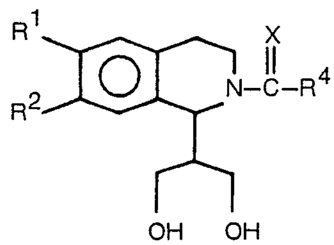

(III),

dans laquelle $R^1$, $R^2$, $R^4$ et X sont tels que définis plus haut, en un dérivé O-acyle correspondant par une migration N→ O-acyle, en présence d'une quantité catalytique d'un acide et,

si cela est désiré, on halogène un composé de formule (IV) ainsi obtenu

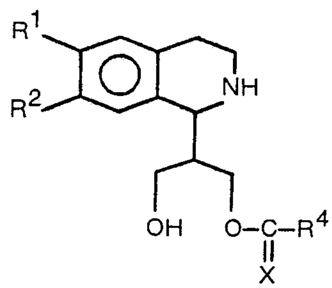

(IV),

dans laquelle $R^1$, $R^2$, $R^4$ et X sont tels que définis plus haut, ou un sel de celui-ci et, si cela est désiré, on coupe le groupe acyle ou thioacyle d'un composé de formule (V):

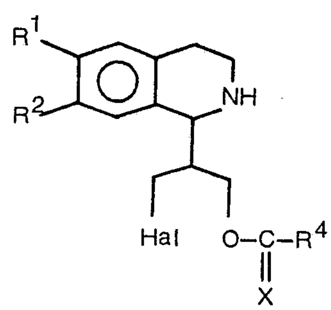

(V),

dans laquelle $R^1$, $R^2$, $R^4$ et X sont tels que définis plus haut et Hal représente un atome d'halogène, ou, si cela est désiré,

on convertit un composé de formule (V) ainsi obtenu dans laquelle $R^1$, $R^2$, $R^4$ et Hal sont tels que définis plus haut, en un composé correspondant de formule (I), dans laquelle $R^3$ représente un groupe

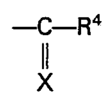

par une migration O→ —N-acyle, dans un milieu alcalin,

et si cela est désiré, on peut détacher à partir des composés dans lesquels $R^7$ et/ou $R^8$ sont des groupes acyles, le ou les groupes acyles par exemple par un traitement à l'acide, pour donner des composés contenant un ou des groupes hydroxy aux positions respectives, et, si cela est désiré, le ou les groupes acyles peuvent être remplacés par d'autres groupes acyles également et/ou, si cela est désiré,

on convertit un composé de formule (I) ainsi obtenu en un sel de celui-ci, ou l'on convertit un sel d'un composé de formule (I) ainsi obtenu en la base libre correspondante de formule (I) et/ou en un autre sel.

2. Procédé suivant la revendication 1, caractérisés en ce que l'halogénation est effectuée avec un halogénure dans la variante $a_1$) du procédé.

3. Procédé suivant la revendication 1, caractérisés en ce que dans la variante $a_2$) du procédé, on emploie un halogénure d'acide ou un anhydride d'acide comme agent acylant convenable pour introduire un groupe

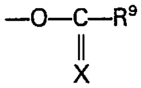

4. Procédé suivant la revendication 1, caractérisés en ce qu'un isocyanate ou un isothiocyanate correspondant est utilisé dans la variante $a_2$) du procédé comme agent acylant convenable pour introduire un groupe

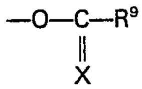

dans lequel $R^9$ est un groupe —NH(alcoyle en $C_{1-4}$) ou NH-phényle.

5. Procédé suivant la revendication 1, caractérisé en ce que dans la variante b) du procédé, un acide minéral, de préférence l'acide chlorhydrique, est utilisé en tant qu'acide.

6. Procédé suivant la revendication 1, caractérisé en ce que des dérivés de [bis(méthyl-substitué)méthyl]-isoquinoléine sont prépararés dans lesquels le groupe alcoyle qui peut être représenté par $R^4$ et/ou $R^9$ ou constituant la partie alcoyle du groupe aralcoyle qui peut être représenté par $R^3$ ou par lequel le groupe phényle, qui peut être représenté par $R^4$ et/ou $R^9$ peut être substitué et/ou le groupe alcoxy qui peut être représenté par $R^1$ et/ou $R^2$ et/ou $R^8$ et/ou le groupe alcoxy par lequel le groupe phényle, qui peut être représenté par $R^4$ et/ou $R^9$, peut être substitué, ont respectivement 1 ou 2 atomes de carbone et la partie alcoyle du groupe —NH (alcoyle en $C_{1-4}$) qui peut être représenté par $R^9$, a 3 ou 4 atomes de carbone.

7. Procèdè suivant la revendication 1, caractérisé en ce que des dérivés de [bis(méthyl-substitué(méthyl]-isoquinoleine sont préparés dans lesquels l'atome ou les atomes d'halogène qui peuvent être représentés par $R^7$ et/ou $R^8$ et/ou par lesquels le groupe phenyle, qui peut être représénté par $R^4$ et/ou $R^9$, peut être substitué, sont respectivement des atomes de chlore et/ou de brome, en particulier de chlore.

8. Procédé suivant la revendication 1, caractérisé en ce que des dérivés de [bis(méthyl-substitué)méthyl]-isoquinoléine sont préparés dans lesquels le groupe cycloalcoyle qui peut être représenté par $R^9$, a 5 ou 6, en particulier 6 atomes de carbone (cyclohexyle).